# FASCICULE DE BREVET EUROPEEN

(11) **EP 1 838 652 B1**
(45) Date de publication et mention de la délivrance du brevet: **25.02.2009**
(21) Numéro de dépôt: 06709087.8
(22) Date de dépôt: 13.01.2006
(51) Int. Cl.: C07C 47/21

(54) **NOUVEAUX COMPOSES ODORANTS, PROCEDE DE SYNTHESE ET UTILISATIONS.**
NEUE DUFTMITTELVERBINDUNGEN SOWIE SYNTHESEVERFAHREN UND VERWENDUNGEN FÜR BESAGTE VERBINDUNGEN
NOVEL ODORANT COMPOUNDS, SYNTHESIS METHOD, AND USES OF SAID COMPOUNDS

(30) Priorité: 19.01.2005 FR 0500551
(43) Date de publication de la demande: 03.10.2007
(73) Titulaire: V. Mane Fils, 06620 Bar sur Loup (FR)
(72) Inventeur: JAUNKY, Piotr, F-06130 Grasse (FR); MANE, Jean, F-06130 Grasse (FR); SCHROEDER, Martin, TN235GE Ashford (GB)
(74) Mandataire: Cabinet Plasseraud
(86) Numéro de dépôt international: PCT/FR2006/000080
(87) Numéro de publication internationale: WO 2006/077305

(56) Documents cités:
- WO-A-01/27234
- US-A- 3 920 752
- US-A- 3 959 396
- PATENT ABSTRACTS OF JAPAN vol. 017, no. 644 (C-1134), 30 novembre 1993 (1993-11-30) & JP 05 202378 A (T HASEGAWA CO LTD), 10 août 1993 (1993-08-10)

## Description

La présente invention concerne de nouveaux composés odorants, qui peuvent être utilisés en parfumerie. L'invention concerne notamment de nouveaux aldéhydes, les alcools et éthers correspondants, leur procédé de synthèse et leurs utilisations en parfumerie.

Le terme parfumerie est ici utilisé pour désigner non seulement la parfumerie au sens habituel du terme, mais également les autres domaines dans lesquels l'odeur des produits est importante. Il peut s'agir de compositions de parfumerie au sens habituel du terme, telles que bases et concentrés parfumants, eaux de Cologne, eaux de toilette, parfums et produits similaires ; de compositions topiques - en particulier cosmétiques - telles que crèmes pour le visage et le corps, poudres de talc, huiles pour cheveux, shampoings, lotions capillaires, sels et huiles de bain, gels de douche et de bain, savons de toilette, antitranspirants et désodorisants corporels, lotions et crèmes de rasage, savons, crèmes, dentifrices, bains de bouche, pommades, et produits similaires ; et de produits d'entretien, tels qu'assouplissants, détergents, lessives, désodorisants d'ambiance, et produits similaires.

Les aldéhydes représentent l'une des grandes familles de molécules odorantes utilisées en parfumerie. Dans cette famille, les aldéhydes possédant entre huit et douze atomes de carbone jouent un rôle important. Parmi ces aldéhydes, on trouve des aldéhydes terpéniques, qui possèdent des odeurs florales. On trouve également des aldéhydes linéaires, dérivés d'acides gras, qui ont des notes fraîches, typiques des fruits de la famille des agrumes.

Le brevet US 3,959,396 décrit des composés à chaîne alkyle, notamment des alcools et des aldéhydes, ayant des propriétés odorante.

Il n'existe pas à ce jour d'aldéhydes combinant ces deux types d'odeur. En tout état de cause, il subsiste un besoin pour de nouveaux agents odorants, afin d'étendre la gamme des notes pouvant être apportées à une composition et les options disponibles pour ajouter ces notes.

La présente invention a pour objet les nouveaux composés représentés par la formule générale (I) suivante : 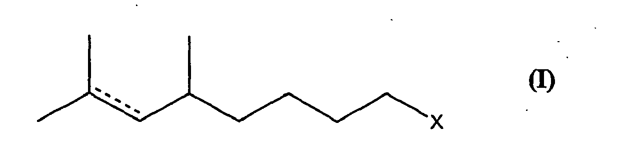
dans laquelle X est un groupe CHO, CH₂OH, CH₂OC(O)R ou CH(OR)₂, et R représente une chaîne alkyle ou alcényle en C1-C5, linéaire ou ramifiée, et
la liaison en pointillé est présente ou absente.

Le composé de formule (I) peut être présent sous forme d'un isomère ou d'un mélange d'isomères, en particulier d'un énantiomère ou d'un mélange d'énantiomères, ou d'un mélange racémique, ou d'un diastéréoisomère ou d'un mélange de diastéréoisomères.

En particulier, la présente invention concerne un composé de formule (I) dans laquelle la liaison en pointillé est présente et X représente un groupe -CHO ; ce nouvel aldéhyde, de formule (1) ci-dessous, est le 6,8-diméthyl-non-7-énal (1). L'invention concerne également le procédé de synthèse et l'utilisation de ce composé (1) comme agent odorant grâce à ses notes fraîches, zestées orange et herbacées aux accents de feuille de coriandre.

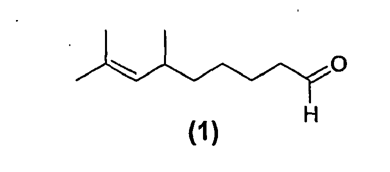

Parmi les énantiomères du composé (1), qui sont tous visés par la présente invention, un composé particulièrement préféré est le 6(R),8-diméthyl-non-7-énal.

La préparation du composé de formule (1) s'effectue, selon l'invention, par hydroformylation du 5,7-diméthyl-octa-1,6-diène, et est représentée par le schéma suivant :

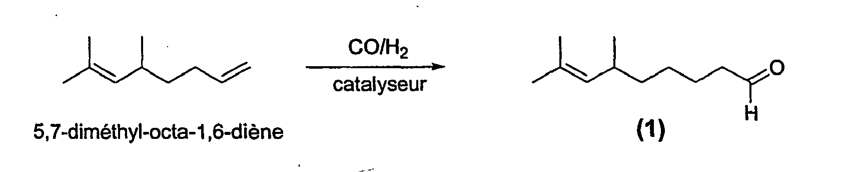

L'hydroformylation est une réaction bien connue. L'homme du métier a aujourd'hui le choix entre plusieurs catalyseurs qui lui permettent d'introduire une fonction aldéhyde dans une molécule insaturée, et sera donc à même de choisir le catalyseur ou système de catalyseurs et de déterminer les conditions de réaction.

A titre d'exemple, dans le procédé selon l'invention, il peut être utilisé un système de catalyseurs, le bis- (η⁴-1,5-cyclooctadiène)-di-µ-méthoxy-di-rhodium(I) (A) et le diphénylphosphino-1,8-diméthyl-9,9-xanthène (Xantphos) (B), décrit par Foça, Claudia; Barros, Humberto J.V. ; dos Santos, Eduardo N. ; Gusevskaya, Elena V. ; Bayon, J.C. ; New Journal of Chemistry (2003), 27(3), 533-539. Ce système de catalyseurs est intéressant du fait de la facilité de sa synthèse et de son utilisation. Il forme en présence de monoxyde de carbone et d'hydrogène un complexe actif, le [RhH(diphosphine)CO₂], qui réagit avec l'alcène présent. D'autres diphosphines, peuvent également être utilisées, pour former un complexe diphosphine-rhodium, comme montré par exemple par del Rio, Immaculada ; de Lange, Wim G.J. ; van Leeuwen, Piet W.N.M. ; Claver, Carmen ; Journal of the Chemical Society, Dalton Transactions (2001), (8), 1293-1300 ou par Dieguez, Montserrat ; Pereira, Mariette M. ; Masdeu-Bulto, Anna M. ; Claver, Carmen ; Bayon, J. Charles ; Journal of Molecular Catalysis A : Chemical (1999), 143-(1-3), 111-112.

Bien sûr, le catalyseur de rhodium, le [Rh (oMe)(cod)₂] (A), peut être également substitué par des catalyseurs comme le [Rh (acac) (co) ₂] ou le [Rh(OAc)(cod)]₂ par exemple, comme décrit par Piet W.N.M. et Carmen Claver (eds) dans « Rhodium Catalysed Hydroformylation » Catalysis by Metal Complexes Vol. 22, Kluwer Academic Publishers, Dordrecht, The Netherlands, (2000), ISBN 0-7923-6551-8.

Suivant un mode de réalisation du procédé selon l'invention, on utilise donc comme catalyseur d'hydroformylation le système de catalyseurs bis-(η⁴-1,5-cyclooctadiène)-di-µ-méthoxy-di-rhodium(I) (A) et diphénylphosphino-1,8-diméthyl-9,9-xanthène (Xantphos) (B). La réaction peut alors être faite en utilisant de 1×10⁻³ à 1×10⁻⁴ équivalent, de préférence de 2×10⁻⁴ à 5×10⁻⁴ équivalents, de catalyseur par rapport à l'alcène de départ.

Suivant ce mode de réalisation, la réaction est faite dans un autoclave dans lequel le 5,7-diméthyl-octa-1,6-diène, bien dégazé, est chargé sous azote. Le catalyseur (A) et le co-catalyseur (B) sont ajoutés également sous azote. La réaction peut être faite en utilisant de 1×¹⁰⁻³ à 1×10⁻⁴ équivalent, de préférence de 2×10⁻⁴ à 5×10⁻⁴ équivalent, de catalyseur par rapport à l'alcène. L'autoclave est ensuite fermé et purgé plusieurs fois avec un mélange équimolaire de monoxyde de carbone et d'hydrogène. La pression intérieure de l'autoclave peut être réglée à une valeur d'environ 5×10³ à 8×10⁴ HPa, de préférence de 3×10⁴ à 6×10⁴ HPa, et le milieu réactionnel est chauffé, notamment à une température d'environ 60 à 120°C, de préférence 100°C. La transformation est suivie par chromatographie gazeuse et le produit est récupéré après environ douze heures et directement distillé en fournissant le 6,8-diméthyl-non-7-énal (1).

La présente invention concerne un autre nouveau composé, le 6,8-diméthyl-non-7-anal (1'), c'est-à-dire le composé de formule (I) où la liaison en pointillé est absente et X représente un groupe -CHO, son procédé de synthèse et son utilisation comme agent odorant.

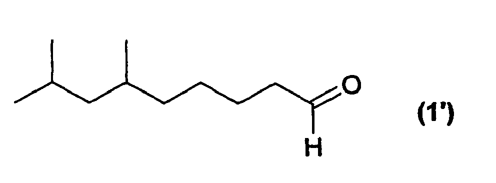

La présente invention concerne également les acétals acycliques ou cycliques des composés (1) et (1'), c'est-à-dire les composés de formule (I) où X représente un groupe -CH(OR)₂ où R représente une chaîne alkyle ou alcényle en C1-C5, linéaire ou ramifiée et la liaison en pointillé est présente ou absente, leur procédé de synthèse et leur utilisation comme agents odorants.

La présente invention concerne aussi l'alcool insaturé (2) et l'alcool saturé (2') dérivés de (1) et (1'), respectivement, c'est-à-dire les composés de formule (I) où X représente un groupe -CH₂OH et la liaison en pointillé est présente (2) ou absente (2'), leur procédé de synthèse et leur utilisation comme agents odorants :

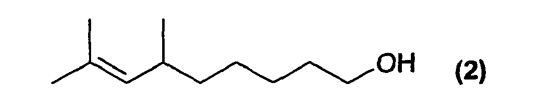

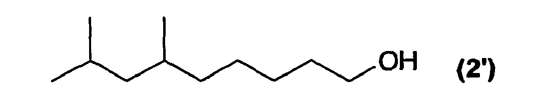

Les alcools (2) et (2') de la présente invention peuvent être aisément préparés par réduction des composés de formule (1) dans des conditions bien connues de l'homme de l'art. Dans un mode de réalisation particulier de l'invention, l'alcool insaturé (2) est obtenu par réduction sélective de la fonction aldéhyde de (1) par un réducteur tel que le borohydrure de sodium (NaBH₄) et l'alcool saturé (2') est synthétisé par réduction de la fonction aldéhyde ainsi que de la double liaison C-C du composé (1) par l'hydrogène en présence d'un catalyseur tel que le palladium sur charbon.

L'aldéhyde saturé (1') de la présente invention peut être préparé par oxydation de (2') selon des méthodes bien connues de l'homme de l'art comme, par exemple, par le réactif de Corey (CrO3/Pyridine/HCl).

La présente invention concerne également les esters (3) et (3'), dérivés des alcools (2) et (2'), respectivement, c'est-à-dire les composés de formule (I) où X représente un groupe -CH₂OC(O)R où R représente une chaîne alkyle ou alcényle C1-C5 linéaire ou branchée et la liaison en pointillé est présente (3) ou absente (3'), en particulier l'acétate, le propionate, le butyrate, l'isobutyrate, le pentanoate, le 2-méthyl-butyrate, le 3-méthyl-butyrate, l'hexanoate, le 2-méthyl-pentanoate, le 3-méthyl-pentanoate, le 4-méthyl-pentanoate, le 2,2'-diméthyl-butyrate, le 2,3-diméthyl-butyrate et le 3,3-diméthyl-butyrate, le 2-butenoate, 2-méthyl-2-butenoate, le 3-hexenoate du 6,8-diméthyl-non-7-énol (2) et du 6,8-diméthyl-non-7-anol (2').

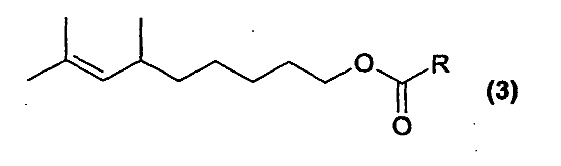

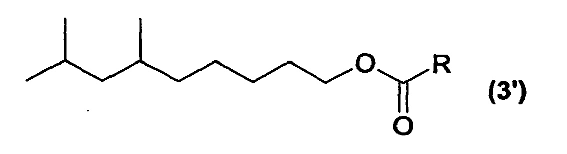

L'invention concerne également le procédé de synthèse de ces esters et leur utilisation comme agents odorants.

Les esters (3) et (3') de la présente invention peuvent être respectivement préparés à partir de (2) et (2'), notamment par estérification des acides ou chlorures d'acyle C1-C5 linéaires ou branchés adéquats, selon des conditions bien connues de l'homme de l'art.

Un schéma de synthèse des composés de l'invention est donné, à titre d'exemple non limitatif, ci-dessous :

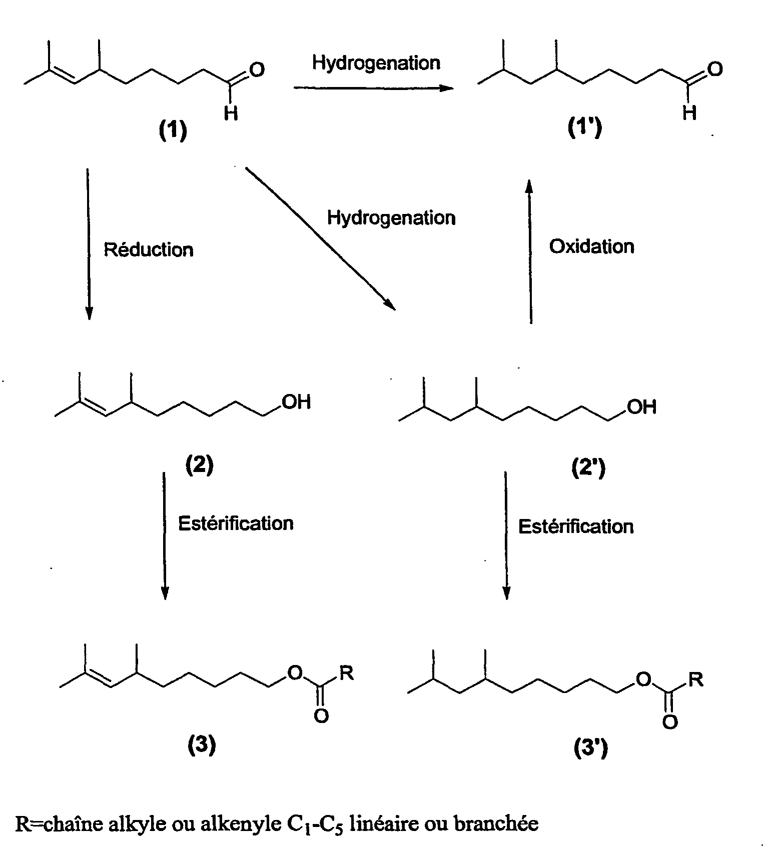

De par leurs propriétés odorantes, les composés de formule (I) trouvent un emploi très varié en parfumerie, au sens détaillé précédemment, notamment et à titre non limitatif en cosmétique et pour les produits d'entretien.

L'invention a également pour objet l'utilisation d'au moins un composé de formule (I) selon l'invention comme agent odorant, comme agent de masquage d'odeur ou comme agent de neutralisation d'odeur, seul ou en en mélange avec un ou plusieurs autres composés odorants connus de l'homme du métier, que l'homme du métier est à même de choisir en fonction de l'effet recherché. Le ou les agents odorants supplémentaires peuvent être des composés de formule (I) ou d'autres agents odorants connus de l'homme du métier.

L'invention a également pour objet les compositions comprenant un produit de base et une quantité efficace d'un ou plusieurs composés de formule (I) selon l'invention.

Il peut s'agir d'une composition elle-même odorante, ou d'une composition dans laquelle l'agent odorant est utilisé pour masquer ou neutraliser certaines odeurs.

Le produit de base sera aisément déterminé par l'homme du métier en fonction de la composition envisagée et donc de l'utilisation envisagée, pour lesquelles les composants habituels, tels que solvant(s) et/ou adjuvant(s), sont bien connus.

La quantité efficace des composés de formule (I) selon l'invention incorporé dans la composition variera selon la nature de la composition, l'effet odorant souhaité, et la nature des autres composés odorants ou non éventuellement présents, et pourra être aisément déterminée par l'homme du métier, sachant qu'elle peut varier dans une plage très étendue, de 0,1 à 99% en poids, en particulier 0,1 à 50% en poids, notamment 0,1 à 30% en poids.

Les composés de formule (I) selon l'invention peuvent être utilisés tels quels ou ils peuvent être incorporés dans ou sur un matériau support inerte ou qui peut contenir d'autres ingrédients actifs de la composition finie. Une grande variété de matériaux supports peut être employée incluant, par exemple, les solvants polaires, les huiles, les graisses, les solides finement divisés, les cyclodextrines, les maltodextrines, les gommes, les résines et n'importe quel autre matériau support connu pour de telles compositions.

L'invention a donc aussi pour objet l'utilisation des composés de formule (I) pour la préparation d'une composition odorante ou d'un article odorant dans les applications décrites ci-dessus, en particulier en parfumerie, en cosmétique, par exemple pour des shampooings ou des savons, et pour des produits d'entretien, tels que des assouplissants ou des lessives.

L'invention concerne en particulier une composition de parfumerie, notamment une base ou un concentré parfumant, une eau de Cologne, une eau de toilette ou un parfum, comprenant au moins un composé de formule (I) ou une composition comprenant au moins un composé de formule (I).

L'invention concerne également en particulier une composition cosmétique, notamment crème pour le visage et le corps, poudre de talc, huile pour cheveux ou pour le corps, shampoing, lotion capillaire, sel de bain, huile de bain, gel de douche, gel de bain, savon de toilette, antitranspirant corporel, désodorisant corporel, lotions, crème de rasage, savon de rasage, crème, dentifrice, bain de bouche, pommade comprenant au moins un composé de formule (I) ou au moins une composition comprenant au moins un composé de formule (I). Un autre objet de l'invention est une méthode de traitement ou de soin cosmétique, préventive ou non, mettant en oeuvre au moins un composé de formule (I) ou au moins une composition comprenant au moins un composé de formule (I).

L'invention concerne encore un produit d'entretien, notamment assouplissant, détergent, lessive, désodorisant d'ambiance, comprenant au moins un composé de formule (I) ou au moins une composition comprenant au moins un composé de formule (I).

Les exemples suivants illustrent davantage les nouveaux composés odorants, leur procédés de synthèse, leur intérêt et leurs utilisations. Ces exemples ne sont présentés que dans un but d'illustration et ne peuvent être considérés comme limitatifs de l'invention.

### Exemple 1 Synthèse du 6,8-diméthyl-non-7-énal (1).

On charge sous azote 138.0 g (1.00 mol) de 5,7-diméthyl-octa-1,6-diène dégazé dans un autoclave, dans lequel un récipient en verre a été placé. On ajoute 232 mg (0.48 mmol) de bis-(η⁴-1,5-cyclooctadiène)-di-µ-méthoxy-di-rhodium(I) (A) et 836 mg (1.44 mmol) de diphénylphosphino-1,8-diméthyl-9,9-xanthène (B). L'auto-clave est fermé et purgé trois fois avec un mélange équimolaire de monoxyde de carbone et d'hydrogène. La pression intérieure est réglée à 3×10⁴ HPa. On chauffe lentement à 100°C, en prenant l'exothermicité de la réaction en considération, sans dépasser 120°C dans la masse. Le milieu réactionnel est chauffé pendant 12 heures sous ces conditions. On refroidit à température ambiante, puis on transfère le produit dans un appareil de distillation. Après avoir récupéré le 5,7-diméthyl-octa-1,6-diène (41.6 g, Téb. : 35°C / 60 HPa), on obtient 88.2 g (0.48 mol) 6,8-diméthyl-non-7-énal (1), Téb. : 48°C / 3.5 HPa). Le rendement est de 48%. Les analyses des spectres infra rouges, RMN et de masse correspondent à la structure de l'aldéhyde (1).

### Exemple 2 Synthèse du 6,8-Diméthyl-non-7-enol (2)

On place dans un ballon de 500 ml muni d'un thermomètre 18 g (0.11 mol) de 6,8-Diméthyl-non-7-enal (1) et 96.0 g d'éthanol. On refroidit à 5°C et on ajoute 2.0 g de borohydrure de sodium en petites portions sans dépasser 10°C dans la masse. Le mélange est agité à température ambiante pendant 16 heures. On refroidit à 5°C et on ajoute goutte à goutte 20.0 g (1.36 mol) d'acétone pour détruire l'excès du réducteur sans dépasser 10°C dans la masse. Le milieu réactionnel est acidifié avec 20.0 g d'acide chlorhydrique à 10% sans dépasser 10°C dans la masse. On ajoute encore 40 g d'eau, puis 70 g de toluène sous forte agitation. Les phases sont séparées et la phase organique est lavée une fois avec 50 g d'eau saturée en NaHCO₃ et deux fois avec 50 g d'eau. On sèche, filtre et évapore le toluène sous pression réduite. On obtient après distillation 13.4 g (0.08 mol) de (Téb. : 105°C/10 Torr). Le rendement est à 72.7%. Les analyses des spectres infra rouges, RMN et de masse correspondent à l'alcool attendu.

### Exemple 3 Acétate du 6,8-Diméthyl-non-7-enol

On place dans un ballon de 500 ml muni d'un thermomètre et d'une ampoule à addition, 13 g (76 mmol) de 6,8-Diméthyl-non-7-enol (2), 10.8 g (107 mmol) de triéthyle amine et 200 ml de t-butyl-méthyl éther (MTBE). On refroidit à 5°C et on ajoute 8.4 g (107 mmol) de chlorure d'acétyle dilué dans 20 ml de MTBE, sans dépasser 10°C dans la masse. Le mélange est agité à température ambiante pendant 14 heures. On refroidit à 5°C et on neutralise avec 100 ml d'une solution à 10% d'acide chlorhydrique. Les phases sont séparées et la phasme organique est lavée une fois avec 100 ml d'eau saturée en NaHCO₃ puis avec de l'eau salée jusqu'à neutralité. On sèche, filtre et évapore le solvant sous pression réduite. L'acétate est purifié par distillation sous pression réduite (Téb. : 58°C/0.4 Torr). Le rendement est de 76%.

### Exemple 4: Propionate du 6,8-Diméthyl-non-7-enol

Il est obtenu selon l'exemple 3 à l'aide de chlorure de propionyle. Le rendement est de 71% (Téb. : 70°C/0.6 Torr).

### Exemple 5: Evaluation olfactive du composé pur (1)

Dans un premier temps, les caractéristiques odorantes du composé (1) pur ont été évaluées par un panel. Le panel d'évaluation est composé de plusieurs professionnels, évaluant qualitativement le composé. Le composé (1) a été décrit comme frais, zesté orange et herbacé aux accents de feuille de coriandre. Si l'aldéhyde (1) est dilué à un pour cent dans le propylène glycol, ses notes fruitées deviennent dominantes et une note de melon peut être perçue.

### Exemple 6: Evaluation olfactive du composé (1) dans deux compositions

Ensuite, deux compositions parfumantes ont été créées, dans lesquelles l'impact olfactif du composé (1) a été examiné. Ces compositions parfumantes peuvent être utilisées pour des produits cosmétiques topiques de type shampoing, gel douche, crème, etc.
Dans chaque cas, les évaluations de l'impact olfactif ont été effectuées à tₒ, t₊₄₈ₕ, t₊₁₆₈ₕ pour évaluer les notes de tête, de coeur et de fond de la composition contenant le composé (1) (essai 2), par comparaison avec une composition ne contenant pas le composé (essai 1).

**Composition n°1**

| Composant | Essai 1 (% en poids) | Essai 2 (% en poids) |
|---|---|---|
| Acétate d'isoamyle | 1.0 | 1.0 |
| Acétate de géranyle | 2.5 | 2.5 |
| Acétate d'hexyle¹⁾ | 1.5 | 1.5 |
| Acétate de styrallyle | 0.5 | 0.5 |
| Acétate de vertényle²⁾ | 2.0 | 2.0 |
| Aldéhyde alpha hexylcinnamique | 10.0 | 10.0 |
| γ-Undécalactone | 4.0 | 4.0 |
| Caproaté d'allyle³⁾ | 1.0 | 1.0 |
| Cyclamen aldéhyde Extra ⁴⁾ | 1.0 | 1.0 |
| Cyclogalbanate⁵⁾ | 0.5 | 0.5 |
| Galaxolide⁶⁾ | 14.0 | 14.0 |
| β-Ionone | 4.0 | 4.0 |
| Méthyl-2-butyrate d'éthyle | 1.5 | 1.5 |
| Terpinéol | 8.0 | 8.0 |
| Triplal⁷⁾ | 0.5 | 0.5 |
| Verdox⁸⁾ | 20.0 | 20.0 |
| Propionate de verdyle⁹⁾ | 2.5 | 2.5 |
| Aldéhyde benzoïque | 2.5 | 2.5 |
| Butyrate d'éthyle¹⁾ | 2.5 | 2.5 |
| Folione¹⁰⁾¹¹⁾ | 3.5 | 3.5 |
| 3-cis-hexènol¹⁾ | 1.5 | 1.5 |
| 6-cis-nonènol¹⁾ | 3.0 | 3.0 |
| Trans-2,cis-6-nonadiènol¹²⁾ | 1.5 | 1.5 |
| Ethyl maltol¹⁾ | 2.5 | 2.5 |
| Dipropylène glycol | 10.0 | 9.0 |
| 6,8-diméthyl-non-7-énal¹⁾ | 0.0 | 1.0 |
| Total | 100.0 | 100.0 |

| | | |
|---|---|---|
| 1. A 10 % dans le dipropylène glycol 2. 4-tert-butylcyclohexyl acétate ; origine : International Flavours and Fragrances, USA. 3. Origine : V. Mane Fils, France. 4. 3-(4-Isopropyl-phenyl)-2-methyl-propionaldehyde ; origine : Givaudan, Suisse. 5. (Cyclohexyloxyl)-acetic acid 2-propenyl ester; origine : Symrise, Allemagne. 6. 1,1,2,3,3,8-Hexamethyl-1,2,3,5,7,8-hexahydro-6-oxa-cyclopenta[b]naphtalène ; origine : International Flavours and Fragrances, USA. 7. 2,4-Dimethyl-cyclohex-3-enecarbaldehyde ; origine : International Flavours and Fragrances, USA. 8. Acetic acid 2-tert-butyl-cyclohexyl ester, origine : International Flavours and Fragrances, USA. 9. Propionic acid 3a,4,5,6,7,7a-hexahydro-1H-4,7-methano-inden-5-yl ester ; origine : Givaudan, Suisse. 10. A 1 % dans le dipropylène glycol 11. Méthyl 2 octinoate ; origine : Givaudan, Suisse. 12. A 1 % dans le dipropylène glycol. | | |

**Composition n° 2**

| Composant | Essai 1 (% en poids) | Essai 2 (% en poids) |
|---|---|---|
| Alcool phényléthylique | 1.0 | 1.0 |
| Aldéhyde alpha hexylcinnamique | 8.0 | 8.0 |
| Cantthoxalc¹⁾ | 0.5 | 0.5 |
| Citronellol | 2.0 | 2.0 |
| Eugénol ²⁾ | 1.0 | 1.0 |
| Galaxolide ³⁾ | 25.0 | 25.0 |
| γ-Décalactone | 0.3 | 0.3 |
| Dihydro jasmonate de méthyle ⁴⁾ | 3.0 | 3.0 |
| Helional ⁵⁾ | 3.0 | 3.0 |
| β-Ionone | 0.2 | 0.2 |
| Isoananate ⁶⁾ | 0.1 | 0.1 |
| ISO E Super ⁷⁾ | 12.0 | 12.0 |
| Lilial ⁸⁾ | 11.0 | 11.0 |
| Linalol | 3.0 | 3.0 |
| Lyral ⁹⁾ | 2.5 | 2.5 |
| Méthylionanthème Super ¹⁰⁾ | 3.0 | 3.0 |
| Rosafix ¹¹⁾ | 0.1 | 0.1 |
| Salicylate d'amyle | 4.0 | 4.0 |
| Salicylate de benzyle | 4.0 | 4.0 |
| Terpinéol | 0.2 | 0.2 |
| Vertenex ¹²⁾ | 4.0 | 4.0 |
| Adoxal ¹³⁾ | 0.4 | 0.4 |
| Aldéhyde phénylacétique ¹⁴⁾ | 0.3 | 0.3 |
| 3(Z)-Hexényte butyrate ¹⁵⁾ | 0.6 | 0.6 |
| Damascénone ¹⁴⁾¹⁶⁾ | 0.2 | 0.2 |
| Indol¹⁴⁾ | 1.2 | 1.2 |
| Melonal¹⁴⁾¹⁷⁾ | 0.8 | 0.8 |
| Targette Ess. Egypte¹⁴⁾ | 2.5 | 2.5 |
| Triplal ¹⁴⁾¹⁸⁾ | 0.8 | 0.8 |
| Dipropylène glycol | 5.3 | 2.8 |
| 6,8-diméthyl-non-7-énal¹⁴⁾ | 0.0 | 2.5 |
| Total | 100.0 | 100.0 |

| | | |
|---|---|---|
| 1.3-(4-Methoxy-phenyl)-2-methyl-propionaldehyde : origine : International Flavours and Fragrances, USA. 2. Origine : V. Mane Fils, France. 3.1,1,2,3,3,8-Hexamethyl-1,2,3,5,7,8-hexahydro-6-oxa-cyclopenta[b]naphtalène ; origine : International Flavours and Fragrances, USA. 4. [3-Oxo-2-((E)-pentyl)-cyclopentyl]-acetic acid methyl ester ; origine : Firmenich, Suisse. 5. 3-Benzo[1,3]dioxol-5-yl-2-methyl-propionaldehyde ; origine : International Flavours and Fragrances, USA. 6. Cyclohexyloxy-acetic acid allyl ester ; origine : Symrise, Allemagne. 7.1-(2,3,8,8-Tetramethyl-1,2,3,4,5,6,7,8-octahydro-naphthalen-2-yl)-ethanone ; origine : International Flavours and Fragrances, USA. 8.3-(4-tert-Butyl-phenyl)-2-methyl-propionaldehyde ; origine : Givaudan, Suisse. 9. 4-(4-Hydroxy-4-methyl-pentyl)-cyclohex-3-enecarbaldehyde, origine : International Flavours and Fragrances, USA. 10. (E)-3-Methyl-4-(2,6,6-trimethyl-cyclohex-2-enyl)-but-3-en-2-one, origine : International Flavours and Fragrances, USA. 11. Acetic acid 2,2,2-trichloro-1-phenyl-ethyl ester ; origine : Symrise, Allemagne. 12. Acetic acid 4-tert-butyl-cyclohexyl ester ; origine : International Flavours and Fragrances, USA. 13. 2,6,10-Trimethyl-undec-9-enal ; origine : Givaudan, Suisse. 14. A 10 % dans le dipropylène glycol. 15. A 1 % dans le dipropylène glycol. 16. 1-(2,6,6-Trimethyl-1,3-cyclohexa-dien-1-yl)-2-butenlone, origine : Firmenich, Suisse. 17. 2,6-Dimethyl-hept-5-enal ; origine : Givaudan, Suisse. 18. 2,4-Dimethyl-cyclohex-3-enecarbaldehyde ; origine : International Flavours and Fragrances, USA. | | |

La présence du 6,8-diméthyl-non-7-énal (1) dans la composition n° 1 change considérablement son odeur d'une pomme à un melon vert, sucré, chair et très agréablement naturel.

Le 6,8-diméthyl-non-7-énal (1) apporte à la composition n° 2 des notes fruitées, notamment du melon vert, ainsi qu'un côté marin. Il donne également plus de puissance aux notes de têtes.

La perte d'intensité avec le temps semble assez linéaire, sans laisser apparaître un important changement de caractéristique odorante.

Les résultats de ces évaluations montrent sans le moindre doute que le composé (1) selon l'invention présente des caractéristiques olfactives intéressantes, qui trouveront une application en parfumerie, en particulier dans les cosmétique et les produits d'entretien, et d'une manière générale dans toute composition odorante ou composition dont il est souhaité de masquer ou neutraliser l'odeur.

### Exemple 7: Evaluation olfactive du composé (2) pur et dans une composition

Un panel d'évaluation similaire à celui de l'exemple 5, a établit, pour le composé (2) pur, la description olfactive suivante : Agreste, floral, bergamote, cinnamique.

L'impact olfactif du composé (2) a ensuite été testé dans une composition bergamote synthétique (essai 2), par comparaison avec une composition ne contenant pas le composé (essai 1).

| | Bergamote sans le 6,8-diméthyl-non-7-énol Essai 1 | Bergamote avec le 6,8-diméthyl-non-7-énol Essai 2 |
|---|---|---|
| Orange terpenes | 270 | 270 |
| Acétate de linalyle | 320 | 320 |
| Linalol | 150 | 150 |
| γ-Terpinène | 75 | 75 |
| β-Pinène | 70 | 70 |
| Citral du Litsea Cubeba | 5 | 5 |
| Orange Brésil ess. | 5 | 5 |
| Acétate de géranyl | 5 | 5 |
| Dipropylène glycol | 100 | X |
| 6,8-diméthyl-non-7-énol | X | 100 |

Les essais effectués montrent que la présence du 6,8-diméthyl-non-7-énol renforce la note bergamote et donne de la puissance au produit. Son effet est encore plus marquant après 2h00 d'évaporation, la note bergamote persiste en coeur.

## Revendications

1. Composé de formule générale (I) : 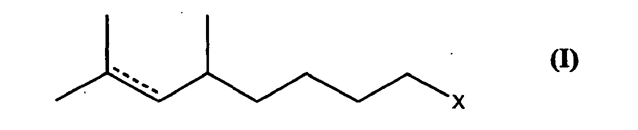 dans laquelle X est un groupe CHO, CH₂OH, CH₂OC(O)R ou CH(OR)₂, et R représente une chaîne alkyle ou alcényle en C1-C5, linéaire ou ramifiée, et
la liaison en pointillé est présente ou absente.

2. Composé selon la revendication 1, **caractérisé en ce qu'**il est sous forme énantiomériquement pure, en particulier sous forme d'énantiomère R ou sous forme d'énantiomère S, ou sous forme de mélange d'énantiomères, en particulier du mélange racémique.

3. Composé selon l'une quelconque des revendications 1 ou 2, **caractérisé en ce qu'**il s'agit du 6,8-diméthyl-non-7-énal (1)

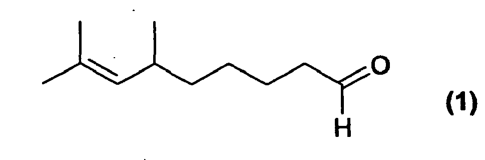

4. Composé selon l'une quelconque des revendications 1 ou 2, **caractérisé en ce qu'**il s'agit du 6(R),8-diméthyl-non-7-énal.

5. Composé selon l'une quelconque des revendications 1 ou 2, **caractérisé en ce qu'**il s'agit du 6,8-diméthyl-non-7-anal (1')

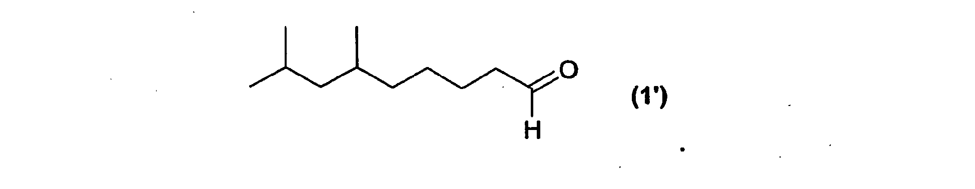

6. Composé selon l'une quelconque des revendications 1 ou 2, **caractérisé en ce qu'**il s'agit du 6,8-diméthyl-non-7-énol (2).

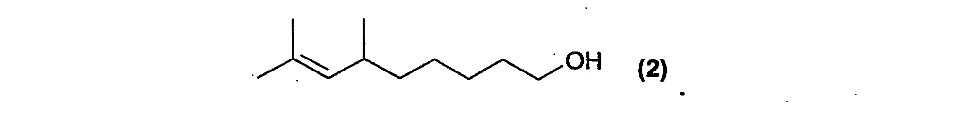

7. Composé selon l'une quelconque des revendications 1 ou 2, **caractérisé en** en ce qu'il s'agit du 6,8-diméthyl-non-7-anol (2')

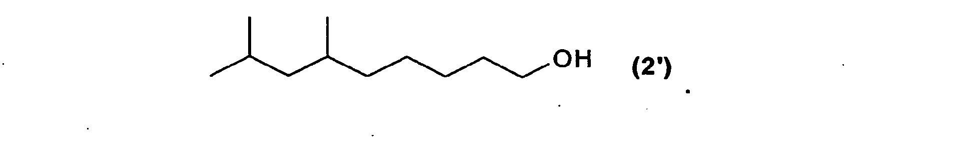

8. Composé selon l'une quelconque des revendications 1 et 2, répondant à formule (I) telle que définie dans la revendication 1, dans laquelle X représente un groupe -CH(OR)₂ où R représente une chaîne alkyle ou alcényle en C1-C5, linéaire ou ramifiée (le groupe -CH(OR)₂ pouvant être cyclique ou acyclique) et la liaison en pointillé est présente ou absente.

9. Composé selon l'une quelconque des revendications 1 et 2, répondant à la formule (I) telle que définie dans la revendication 1, dans laquelle X représente un groupe -CH₂OC(O) où R représente une chaîne alkyle ou alcényle C1-C5 linéaire ou branchée et la liaison en pointillé est présente (3) ou absente (3')

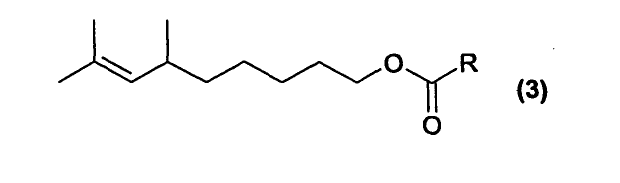

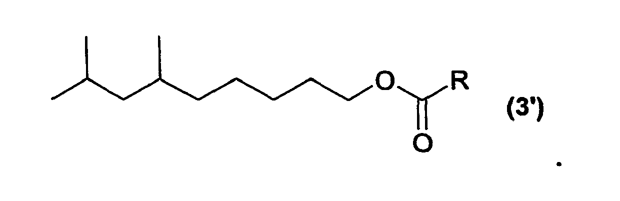

10. Composé selon la revendication 9, qui est un ester dérivé des alcools (2) et (2') définis dans les revendications 6 et 7, et notamment l'acétate, le propionate, le butyrate, l'isobutyrate, le pentanoate, le 2-méthyl-butyrate, le 3-méthyl-butyrate, l'hexanoate, le 2-méthyl-pentanoate, le 3-méthyl-pentanoate, le 4-méthyl-pentanoate, le 2,2-diméthyl-butyrate, le 2,3-diméthyl-butyrate et le 3,3-diméthyl-butyrate, le 2-butenoate, 2-méthyl-2-butenoate, le 3-hexenoate du 6,8-diméthyl-non-7-énol (2) et du 6,8-diméthyl-non-7-anol (2').

11. Procédé de préparation du composé de formule (1) : 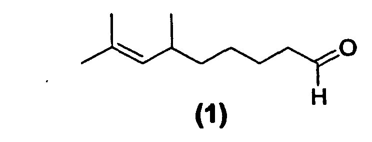 comprenant l'hydroformylation du 5,7-diméthyl-octa-1,6-diène.

12. Procédé selon la revendication 11, **caractérisé en ce qu'**on utilise le système de catalyseurs bis-(η⁴-1,5-cyclooctadiène)-di-µ-méthoxy-di-rhodium (I) et diphénylphosphino-1,8-diméthyl-9,9-xanthène (Xantphos) en tant que catalyseur de l'hydroformylation.

13. Composition **caractérisée en ce qu'**elle contient au moins un composé de formule (I), tel que défini dans l'une quelconque des revendications 1 à 10, sous forme d'un isomère ou d'un mélange d'isomères, en particulier d'un énantiomère ou d'un mélange d'énantiomères, ou d'un mélange racémique, ou d'un diastéréoisomère ou mélange de diastéréoisomères.

14. Composition selon la revendication 13, **caractérisée en ce que** le ou lesdits composés de formule (I) sont incorporés dans ou sur un matériau support inerte ou qui peut contenir d'autres ingrédients actifs, ledit support étant notamment choisi parmi les solvants polaires, les huiles, les graisses, les solides finement divisés, les cyclodextrines, les maltodextrines, les gommes, les résines.

15. Composition de parfumerie, notamment base ou concentré parfumant, eau de Cologne, eau de toilette ou parfum, **caractérisée en ce qu'**elle comprend au moins un composé tel que défini dans l'une quelconque des revendications 1 à 10 ou une composition selon l'une quelconque des revendications 13 ou 14.

16. Composition cosmétique, notamment crème pour le visage et le corps, poudre de talc, huile pour cheveux ou pour le corps, shampoing, lotion capillaire, sel de bain, huile de bain, gel de douche, gel de bain, savon de toilette, antitranspirant corporel, désodorisant corporel, lotions, crème de rasage, savon de rasage, crème, dentifrice, bain de bouche, pommade, **caractérisée en ce qu'**elle comprend au moins un composé tel que défini dans l'une quelconque des revendications 1 à 10 ou une composition selon l'une quelconque des revendications 13 ou 14.

17. Produit d'entretien, notamment assouplissant, détergent, lessive, désodorisant d'ambiance, **caractérisé en ce qu'**il comprend au moins un composé tel que défini dans l'une quelconque des revendications 1 à 10 ou une composition selon l'une quelconque des revendications 13 ou 14.

18. Utilisation d'au moins un composé de formule (I) tel que défini dans l'une quelconque des revendications 1 à 10, ou d'une composition selon l'une quelconque des revendications 13 ou 14, comme agent odorant ou comme agent de masquage d'odeur ou comme agent de neutralisation d'odeur.

19. Utilisation selon la revendication 18, dans lequel le(s)dit(s) composé ou ladite composition est utilisé(e) en association avec d'autres agents odorants.

## Claims

1. A compound of general formula (I): 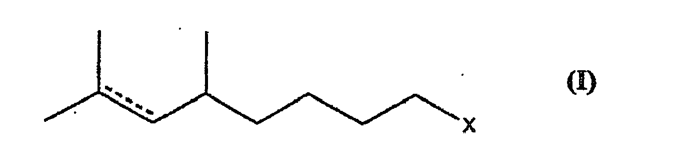 in which X is a CHO, CH₂OH, CH₂OC(O)R or CH(OR)₂ group and R represents a linear or branched C₁-C₅ alkyl or alkenyl chain, and
the bond given as a dotted line is present or absent.

2. The compound as claimed in claim 1, **characterized in that** it is in the enantiomerically pure form, in particular in the form of the R enantiomer or in the form of the S enantiomer, or in the form of a mixture of enantiomers, in particular of the racemic mixture.

3. The compound as claimed in either one of claims 1 and 2, **characterized in that** it is 6,8-dimethylnon-7-enal (1)

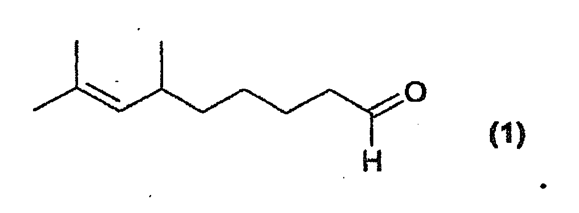

4. The compound as claimed in either one of claims 1 and 2, **characterized in that** it is 6(R),8-dimethylnon-7-enal.

5. The compound as claimed in either one of claims 1 and 2, **characterized in that** it is 6,8-dimethylnon-7-anal (1')

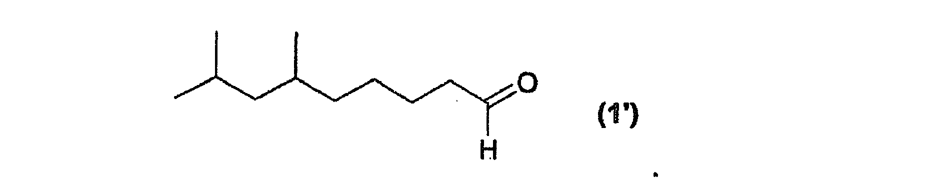

6. The compound as claimed in either one of claims 1 and 2, **characterized in that** it is 6,8-dimethylnon-7-enol (2).

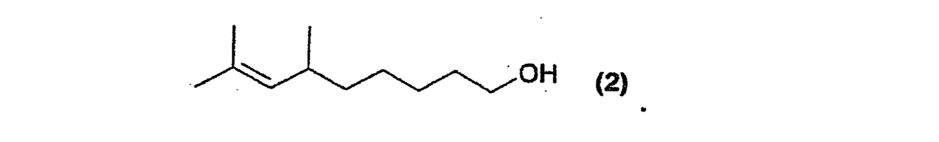

7. The compound as claimed in either one of claims 1 and 2, **characterized in that** it is 6,8-dimethylnon-7-anol (2')

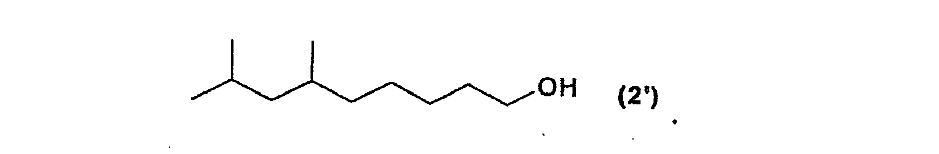

8. The compound as claimed in either one of claims 1 and 2, corresponding to the formula (I) as defined in claim 1 in which X represents a -CH(OR)₂ group where R represents a linear or branched C₁-C₅ alkyl or alkenyl chain (it being possible for the - CH(OR)₂ group to be cyclic or acyclic) and the bond given as a dotted line is present or absent.

9. The compound as claimed in either one of claims 1 and 2, corresponding to the formula (I) as defined in claim 1 in which X represents a -CH₂OC(O)R group where R represents a linear or branched C₁-C₅ alkyl or alkenyl chain and the bond given as a dotted line is present (3) or absent (3')

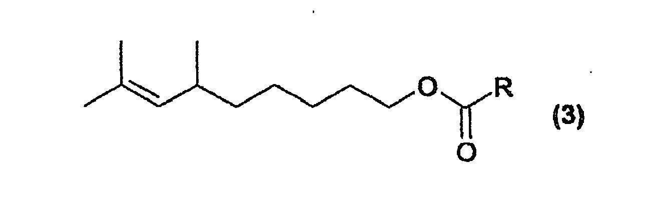

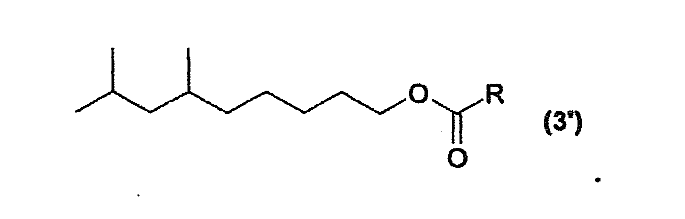

10. The compound as claimed in claim 9, which is an ester derived from the alcohols (2) and (2') defined in claims 6 and 7 and in particular the acetate, propionate, butyrate, isobutyrate, pentanoate, 2-methylbutyrate, 3-methylbutyrate, hexanoate, 2-methylpentanoate, 3-methylpentanoate, 4-methylpentanoate, 2,2-dimethylbutyrate, 2,3-dimethylbutyrate and 3,3-dimethylbutyrate, 2-butenoate, 2-methyl-2-butenoate or 3-hexenoate of 6,8-dimethylnon-7-enol (2) and 6,8-dimethylnon-7-anol (2').

11. A process for the preparation of the compound of formula (I): 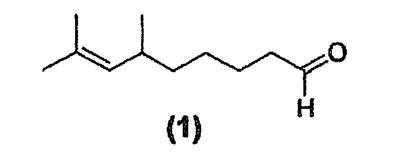 comprising the hydroformylation of 5,7-dimethylocta-1,6-diene.

12. The process as claimed in claim 11, **characterized in that** use is made of the system of catalysts bis(η⁴-1,5-cyclooctadiene)di(µ-methoxy)di-rhodium(I) and diphenylphosphino-1,8-dimethyl-9,9-xanthene (Xantphos) as catalyst for the hydroformylation.

13. A composition, **characterized in that** it comprises at least one compound of formula (I) as defined in any one of claims 1 to 10 in the form of an isomer or of a mixture of isomers, in particular of an enantiomer or of a mixture of enantiomers, or of a racemic mixture, or of a diastereoisomer or mixture of diastereoisomers.

14. The composition as claimed in claim 13, **characterized in that** said compound or compounds of formula (I) are incorporated in or on an inert support material or a support material which can comprise other active ingredients, said support being chosen in particular from polar solvents, oils, fats, finely divided solids, cyclodextrins, maltodextrins, gums or resins.

15. A perfumery composition, in particular fragrance base or perfume concentrate, eau de Cologne, toilet water or fragrance, **characterized in that** it comprises at least one compound as defined in any one of claims 1 to 10 or a composition as claimed in either one of claims 13 and 14.

16. A cosmetic composition, in particular a cream for the face and body, talcum powder, oil for the hair or for the body, shampoo, hair lotion, bath salt, bath oil, shower gel, bath gel, toilet soap, antiperspirant for the body, deodorant for the body, shaving lotion or cream, shaving soap, cream, toothpaste, mouthwash or pomade, **characterized in that** it comprises at least one compound as defined in any one of claims 1 to 10 or a composition as claimed in either one of claims 13 and 14.

17. A cleaning product, in particular softener, detergent, washing powder or air freshener, **characterized in that** it comprises at least one compound as defined in any one of claims 1 to 10 or a composition as claimed in any one of claims 13 and 14.

18. The use of at least one compound of formula (I) as defined in any one of claims 1 to 10 or of a composition as claimed in either one of claims 13 and 14 as odorous agent or as odor-masking agent or as odor-neutralizing agent.

19. The use as claimed in claim 18, in which said compound(s) or said composition is used in combination with other odorous agents.

## Patentansprüche

1. Verbindung der allgemeinen Formel (I), 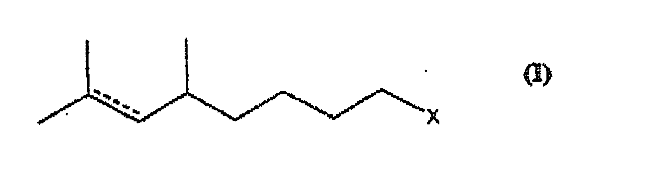 in der X eine CHO-, CH₂OH-, CH₂OC(O)R- oder CH(OR)₂-Gruppe bedeutet und R eine geradkettige oder verzweigte C1-C5-Alkyl- oder- Alkenylkette bedeutet, und
die gepunktet eingezeichnete Bindung vorhanden ist oder fehlt.

2. Verbindung nach Anspruch 1, **dadurch gekennzeichnet, daß** sie in enantiomerenreiner Form, insbesondere in Form eines R-Enantiomers oder in Form eines S-Enantiomers, oder in Form einer Enantiomerenmischung, insbesondere in Form einer racemischen Mischung, vorliegt.

3. Verbindung nach einem der Ansprüche 1 oder 2, **dadurch gekennzeichnet, daß** es sich um 6,8-Dimethylnon-7-enal (1) 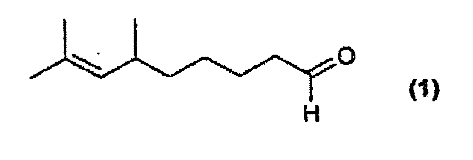 handelt.

4. Verbindung nach einem der Ansprüche 1 oder 2, **dadurch gekennzeichnet, daß** es sich um 6(R)-,8-Dimethylnon-7-enal handelt.

5. Verbindung nach einem der Ansprüche 1 oder 2, **dadurch gekennzeichnet, daß** es sich um 6,8-Dimethylnon-7-anal (1') 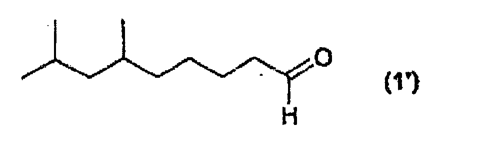 handelt.

6. Verbindung nach einem der Ansprüche 1 oder 2, **dadurch gekennzeichnet, daß** es sich um 6,8-Dimethylnon-7-enol (2) 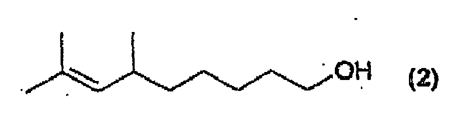 handelt.

7. Verbindung nach einem der Ansprüche 1 oder 2, **dadurch gekennzeichnet, daß** es sich um 6,8-Dimethylnon-7-anol (2') 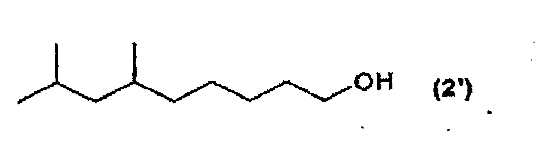 handelt.

8. Verbindung nach einem der Ansprüche 1 und 2, die der Formel (I) wie in Anspruch 1 definiert entspricht, wobei X eine Gruppe - CH(OR)₂ bedeutet, wobei R eine geradkettige oder verzweigte C1-C5-Alkyl- oder -Alkenylkette bedeutet (wobei die Gruppe -CH(OR)₂ zyklisch oder nicht-zyklisch sein kann) und die gepunktet eingezeichnete Bindung vorhanden ist oder fehlt.

9. Verbindung nach einem der Ansprüche 1 und 2, die der Formel (I) wie in Anspruch 1 definiert entspricht, wobei X eine Gruppe - CH₂OC(O)R bedeutet, wobei R eine geradkettige oder verzweigte C1-C5-Alkyl- oder -Alkenylkette bedeutet und die gepunktet eingezeichnete Bindung vorhanden ist (3) oder fehlt (3').

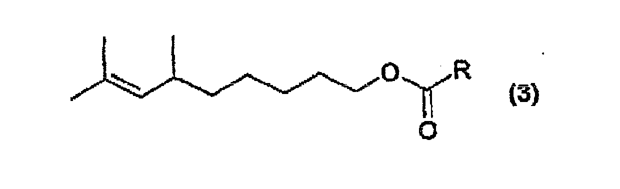

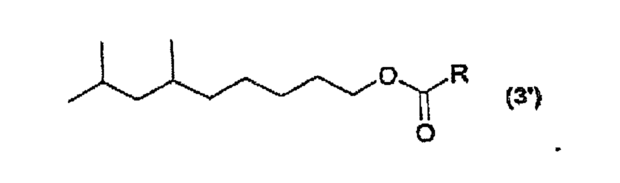

10. Verbindung nach Anspruch 9, wobei es sich um ein Esterderivat der in den Ansprüchen 6 und 7 definierten Alkohole (2) und (2') handelt, insbesondere um das Acetat, Propionat, Butyrat, Isobutyrat, Pentanoat, 2-Methylbutyrat, 3-Methylbutyrat, Hexanoat, 2-Methylpentanoat, 3-Methylpentanoat, 4-Methylpentanoat, 2,2-Dimethylbutyrat, 2,3-Dimethylbutyrat und 3,3-Dimethylbutyrat, 2-Butenoat, 2-Methyl-2-butenoat, 3-Hexenoat des 6,8-Dimethylnon-7-enols (2) und des 6,8-Dimethylnon-7-anols (2').

11. Verfahren zur Herstellung der Verbindung der Formel (1) 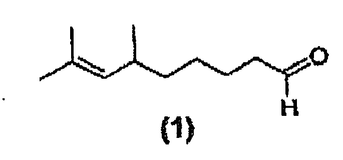 wobei 5,7-Dimethylocta-1,6-dien hydroformyliert wird.

12. Verfahren nach Anspruch 11, **dadurch gekennzeichnet, daß** man das Katalysatorsystem Bis-(η⁴-1,5-cyclooctadien)-di-µ-methoxydirhodium (I) und Diphenylphosphino-1,8-dimethyl-9,9-xanthen (Xantphos) als Hydroformylierungskatalysator verwendet.

13. Zusammensetzung, **dadurch gekennzeichnet, daß** sie mindestens eine Verbindung der Formel (I) wie in einem der Ansprüche 1 bis 10 definiert in Form eines Isomers oder einer Isomerenmischung, insbesondere eines Enantiomers oder einer Enantiomerenmischung oder einer racemischen Mischung oder eines Diastereomers oder einer Diastereomerenmischung enthält.

14. Zusammensetzung nach Anspruch 13, **dadurch gekennzeichnet, daß** die Verbindung(en) der Formel (I) in oder auf ein inertes Trägermaterial oder ein Trägermaterial, das andere Wirkstoffe enthalten kann, eingebaut sind, wobei der genannte Träger insbesondere aus der Reihe der polaren Lösungsmittel, Öle, Fette, feinteiligen Feststoffe, Cyclodextrine, Maltodextrine, Gummis und Harze stammen kann.

15. Parfümzusammensetzung, insbesondere Parfümbasis oder - konzentrat, Eau de Cologne, Eau de Toilette oder Parfüm, **dadurch gekennzeichnet, daß** sie mindestens eine Verbindung, wie in einem der Ansprüche 1 bis 10 definiert, oder eine Zusammensetzung nach einem der Ansprüche 13 oder 14 enthält.

16. Kosmetikzusammensetzung, insbesondere Gesichts- und Körpercreme, Talkumpuder, Haar- oder Körperöl, Shampoo, Haarlotion, Badesalz, Badeöl, Duschgel, Badegel, Toilettseife, Körperantitranspirant, Körperdeodorant, Lotionen, Rasiercreme, Rasierseife, Creme, Zahncreme, Mundwasser, Pomade, **dadurch gekennzeichnet, daß** sie mindestens eine Verbindung, wie in einem der Ansprüche 1 bis 10 definiert, oder eine Zusammensetzung nach einem der Ansprüche 13 oder 14 enthält.

17. Pflegeprodukt, insbesondere geschmeidigmachendes Produkt, Reinigungsmittel, Waschmittel, Umweltdeodorant, **dadurch gekennzeichnet, daß** es mindestens eine Verbindung, wie in einem der Ansprüche 1 bis 10 definiert, oder eine Zusammensetzung nach einem der Ansprüche 13 oder 14 enthält.

18. Verwendung von mindestens einer Verbindung der Formel (I) nach einem der Ansprüche 1 bis 10 oder einer Zusammensetzung nach einem der Ansprüche 13 oder 14 als Duftstoff oder als Geruchsmaskierungsmittel oder als Geruchsneutralisierungsmittel.

19. Verwendung nach Anspruch 18, wobei die genannte(n) Verbindung(en) oder die genannte Zusammensetzung in Kombination mit anderen Duftstoffen verwendet wird/werden.
